# EUROPEAN PATENT SPECIFICATION

(11) **EP 0 882 009 B1**
(45) Date of publication and mention of the grant of the patent: **16.05.2001**
(21) Application number: 97903489.9
(22) Date of filing: 18.02.1997
(51) Int. Cl.: C07C 63/26, C07C 51/43

(54) **PRODUCTION OF AROMATIC POLYCARBOXYLIC ACIDS**
HERSTELLUNG VON AROMATISCHEN POLYCARBONSÄUREN
PRODUCTION D'ACIDES POLYCARBOXYLIQUES AROMATIQUES

(30) Priority: 22.02.1996 GB 9603740; 29.08.1996 GB 9617994; 09.12.1996 US 33276 P
(43) Date of publication of application: 09.12.1998
(73) Proprietor: E.I. DU PONT DE NEMOURS AND COMPANY, Wilmington Delaware 19898 (US)
(72) Inventor: BICKHAM, David, Robert, Guisborough, Cleveland TS14 7LX (GB); JEFFERY, Ian, Charles, Stockton-on-Tees, Cleveland TS20 1LP (GB); WHISTON, Keith, Darlington, County Durham DL3 8EA (GB); WATSON, Brian, Robert, Saltburn, Cleveland TS12 2PX (GB)
(74) Representative: Jones, Alan John
(86) International application number: GB9700439
(87) International publication number: WO9730963

(56) References cited:
- EP-A- 0 502 628
- WO-A-93/24440

## Description

This invention relates to the production of aromatic carboxylic acids, especially dicarboxylic acids such as terephthalic acid.

Terephthalic acid for example is produced commercially by oxidising p-xylene with oxygen in a liquid phase which comprises a lower aliphatic carboxylic acid solvent, such as acetic acid, and a dissolved heavy metal catalyst system (usually cobalt and manganese and a bromine promoter). A slurry of terephthalic acid in the solvent is withdrawn from the reactor and is subjected to a solids-liquid separation process resulting in crude terephthalic acid crystals which may be subsequently processed further and a mother liquor filtrate which, in addition to catalyst and promoter used in the oxidation reaction, contains dissolved terephthalic acid and various by-products and impurities. These by-products and impurities arise from various sources such as minor impurities in the p-xylene feed stock to the reaction, incomplete oxidation of p-xylene resulting in partially oxidised products and by-products arising from the competing side reactions in the oxidation of p-xylene to terephthalic acid.

It is common practice to recycle a large proportion of the recovered mother liquor to the oxidation reaction in order to return catalyst and promoter to the oxidation reaction while purging a smaller proportion to a solvent recovery system so as to maintain the level of impurities and by-products in the reaction within tolerable limits, see, e.g., Kirk -Othmer Encyclopedia of Chemical Technology, 3rd Edition, 1979, vol. 17, page 749 and US-A-4 939 297, in the solvent recovery system, the mother liquor purge is subjected to evaporation to remove a substantial proportion of the aliphatic acid solvent and water present (which can be returned to the oxidation reaction) leaving a concentrate containing terephthalic acid and impurities/by-products together with some of the heavy metal catalyst present in the original mother liquor filtrate. The concentrate (the residues) may then be disposed of or, if economically justifiable, treated in order to recover valuable components for recycling, e.g. catalyst metals. Typical downstream treatments of the residues include catalyst recovery, incineration and anaerobic/aerobic biological treatment to reduce chemical oxygen demand (COD).

The present invention is concerned with a process for the production of an aromatic carboxylic acid comprising oxidising a precursor of the aromatic carboxylic acid in an aqueous liquid phase medium comprising acetic acid and in the presence of a heavy metal catalyst system, withdrawing from the reaction mixture a slurry of the aromatic carboxylic acid in mother liquor comprising mainly the acetic acid, subjecting the slurry to a solids-liquid separation to recover crystals of aromatic carboxylic acid, recycling a first fraction of the resulting mother liquor to the oxidation reaction, concentrating a second fraction of the separated mother liquor to remove acetic, and disposing of or processing the concentrated residue. Such a process is hereinafter referred to as being "of the kind specified".

According to the present invention there is provided a process of the kind specified in which the second fraction of mother liquor is subjected to solids-liquid separation prior to concentration thereof.

As in conventional practice, the first fraction will usually be substantially larger than the second fraction.

By subjecting the second mother liquor fraction to solids-liquid separation prior to concentration thereof, we have found that a small but useful amount of the aromatic carboxylic acid can be recovered from the mother liquor since some aromatic carboxylic acid in the form of fine particles tends to pass through the solids-liquid separation with the mother liquor. The solids-liquid separation treatment is conveniently carried out so as to remove at least a major proportion of the fines present in the second mother liquor fraction.

Typically the size range of particles removed is 10 to 100 microns. The aromatic carboxylic acid concentration in mother liquor is typically 0.5 to 1.5% by weight and 50 to 80% of these solids are recovered.

Although reference is made to first and second fractions, we do not exclude the possibility of the mother liquor being split into more than two fractions provided that at least one fraction is returned to the oxidation reaction and at least one fraction is treated to remove said fines.

By removing the aromatic carboxylic acid in this manner, the organic content of the second mother liquor fraction can be reduced significantly thereby reducing the load on downstream equipment used to concentrate the second mother liquor fraction and treat or dispose of the resulting residue.

In particular, the reduced amount of organic material in the feed to the concentrating equipment such as an evaporator leads to an improvement in performance of such equipment thereby allowing use of smaller equipment or affording potential uprating of existing plant. During concentration of the second mother liquor fraction, the acetic acid is driven off. Because the evaporator has to handle less solids content of a different compositional nature when the process of the present invention is employed, it becomes possible to recover more of the acetic acid component since the amount of liquid necessary to maintain fluidity in the evaporator bottoms is reduced. Typically, acetic acid levels in reslurried evaporator bottoms fall from between 10 to 15% to 3 to 7%. In this context, it will be understood that it is important to maintain fluidity of the evaporator bottoms otherwise a mass having poor flow characteristics would result with consequent difficulties in handling.

Where the concentrated residue is subjected to incineration the load on such equipment is likewise reduced by the reduction in the organic content of the second mother liquor fraction, making downsizing of the incinerator possible. Similarly, where the concentrated residue is subjected to further processing such as catalyst recovery or biological treatment, the load on the equipment employed is reduced. Where the concentrated residue is simply sent to landfill, the amount to be disposed is reduced.

Moreover, the process of the invention allows the recovery of aromatic carboxylic acid which would otherwise be lost in a waste stream.

The aromatic carboxylic acid recovered from the second fraction may be recycled to the oxidation reaction, for example by reslurrying it in solvent and returning the resulting slurry to the oxidation process. Thus, for example the aromatic carboxylic acid may be reslurried with mother liquor comprising said first fraction and/or with solvent recovered during concentration of the second fraction. Alternatively, the aromatic carboxylic acid recovered from the second fraction may be transferred to the next stage of processing along with terephthalic acid recovered from the slurry in the solids-liquid separation. Such further stage of processing may comprise purification for example by hydrogenation of an aqueous solution of crude aromatic carboxylic acid as disclosed for instance in European Patent Applications Nos. 498591 and 502628 or International Patent Applications Nos. WO93/24440 and WO94/17892 (the entire disclosures of which are incorporated herein by this reference).

In another embodiment, at least some of the aromatic carboxylic acid recovered from the second fraction may be introduced into a crystallisation process following the oxidation reaction and preceding the primary solids-liquid separation (separating crude terephthalic acid and the mother liquor) in order to secure growth of the fines to a larger particle size. The crystallisation process serves to induce further precipitation of the aromatic carboxylic acid from solution in the mother liquor by reducing pressure and temperature in one or more crystallisation vessels.

Where the recovered aromatic carboxylic acid is recycled to the oxidation reaction it may be combined with aromatic carboxylic acid recovered from elsewhere in the process and the combined amounts of aromatic carboxylic acid may be reslurried with mother liquor and/or recovered solvent for recycle to the reaction as a slurry. For example, in our prior European Patent Application No. 498591, we disclose a process for the purification of crude aromatic carboxylic acid in which, following purification and separation of the purified aromatic carboxylic acid crystals from aqueous mother liquor, the mother liquor is further treated to precipitate less pure aromatic carboxylic acid which is recycled to the oxidation process. Thus, in the present invention, less pure aromatic carboxylic acid derived from a purification process for terephthalic acid or other appropriate aromatic carboxylic acid may be combined with aromatic carboxylic acid recovered from the second fraction of the oxidation mother liquor and returned to the oxidation reaction.

The solvent, eg acetic acid, recovered from the concentration of the second mother liquor fraction may be transferred to facilities for storage, disposal and/or further processing or it may be recycled to the oxidation reaction. The concentrated residue may be disposed of, for example by incineration or landfill, or subjected to further processing to recover valuable constituents thereof.

The primary solids-liquid separation will usually be preceded by a crystallisation process, i.e. the slurry obtained from the oxidation reaction will usually be passed through one or more stages of crystallisation to promote further precipitation of the aromatic carboxylic acid from solution thereby increasing the yield of aromatic carboxylic acid crystals obtained from the solids-liquid separation.

The primary solids-liquid separation may be carried out using conventional techniques but preferably it is carried out in a pressurised integrated filtration and washing apparatus as disclosed in European Patent Application No. 502628 or International Patent Application No. WO94/17892 preferably with a countercurrent wash using water as the wash liquor so that the liquid content of the filtered and washed aromatic carboxylic acids obtained comprises water, i.e. the solvent used in the purification stage of processing where this is employed. Preferably the integrated filtration and washing apparatus comprises belt filtration equipment operating under elevated pressure conditions. Alternative forms of filtration and washing apparatus include rotary filters such as rotary filters in which a pressure differential is established between the slurry and filtrate sides of a cylindrical filter medium to effect displacement of mother liquor and wash water through the filter cake, and pressure drum filters in which the mother liquor is displaced from the filter cake by water under hydraulic pressure (as in for example a BHS-Fest pressurised drum filter).

The technique used to effect solids-liquid separation of the second mother liquor fraction is selected to ensure adequate recovery of the aromatic carboxylic acid fines present. A preferred technique is filtration, conveniently using a filter in which build up of a filter cake on a filter medium occurs in such a way that the filter cake is itself instrumental in filtering the solids-containing liquid. Various forms of filtration processes are suitable, e.g. a pressure vessel equipped with a candle filter or filters.

After concentration of the second mother liquor fraction, the resulting residues may be processed using conventional techniques such as incineration and/or biological treatment (anaerobic and/or aerobic).

However, in a preferred aspect of the invention, the concentrated residue including a substantial part of its organic content is dissolved in an aqueous medium and the metal catalyst components are precipitated from the solution.

The metal catalyst components may be precipitated from said solution by the inclusion of metal salt-forming anions in the solution.

Typically the aromatic carboxylic acid is one which has very low solubility in water, viz. less than 1% by weight at 25°C, e.g. terephthalic acid.

Preferably substantially the whole of the residue is dissolved in said aqueous medium. Conveniently the catalyst metals are precipitated from the aqueous medium by the addition of carbonate and/or bicarbonate ions to the medium. By dissolving substantially the whole of the residue, the catalyst metal yield can be increased since catalyst metals occluded, chemically or otherwise, with the organics are taken into solution and can then be precipitated, for example as carbonates and/or bicarbonates.

Because aromatic carboxylic acid is removed from the second mother liquor fraction prior to concentration, not only can the organic content of the second mother liquor fraction be reduced significantly with consequent reduction of the load on downstream equipment used to concentrate the second mother liquor fraction but the organic content of the resulting concentrated residue is significantly reduced by elimination of the aromatic carboxylic acid which may have low solubility in water thus making it feasible to carry out residue treatment involving dissolution of substantially the whole of the residue in said aqueous medium.

After precipitation of the catalyst metals, the residual liquor may be processed using conventional techniques such as biological treatment (anaerobic and/or aerobic).

Precipitation of the metal catalysts is conveniently effected by inclusion in the aqueous medium of the carbonate and/or bicarbonate reaction product obtained following contacting a metal or ammonium hydroxide with a carbon dioxide-containing offgas derived from the oxidation reaction in which said polycarboxylic acid is produced.

Preferably the aqueous medium comprises, as at least a major component thereof, an organic material-containing mother liquor derived from the hydrogenation of an aqueous solution of the polycarboxylic acid. In contrast with prior catalyst recovery schemes in which water is used to extract the desired metals from the residue, the process of the present invention involves the solubilisation of substantially the whole of the residue before precipitating the catalyst metals, thus making it feasible to use an organics containing aqueous medium for dissolution of the metals and organic content of the residue.

Solubilisation of substantially all of the residue in the aqueous medium may be effected by inclusion of an alkaline agent added to the aqueous medium prior to and/or in the course of combining the residue with the aqueous medium. The agent may comprise ammonium hydroxide or a metal hydroxide, such as sodium hydroxide. Alternatively , pH may be increased initially by the inclusion of carbonate and/or bicarbonate ions in the aqueous medium either as the sole alkaline agent or in combination with another alkaline agent or agents such as ammonium hydroxide or a metal hydroxide. The inclusion of carbonate and/or bicarbonate ions in the aqueous medium (by addition to the aqueous medium prior to or after contacting the same with the residue) is considered advantageous since the metal recovered in the form of carbonates and/or bicarbonates is of a higher quality, apparently because less oxide contamination occurs compared with use of hydroxide only in the initial solubilisation of the residue. For this reason, the process of the invention may with advantage be carried out using carbonate and/or bicarbonate ions as the major or sole alkaline agent in effecting initial solubilisation of the residue.

Typically the alkaline agent is introduced to raise the pH sufficiently, preferably at least to 5.5, to neutralise the acidic content of the residue (and aqueous medium where the latter contains acidic components as in the case where it is constituted by the mother liquor derived from said hydrogenation reaction) partially and dissolve the same. Subsequently carbonate and/or bicarbonate reaction ions are added to raise the pH further to precipitate the catalyst metals and secure a pH compatible with downstream processing of the liquor remaining following separation of the solids. For instance, the pH is conveniently increased to about 6.5 to about 9, preferably about 7 to 8, by addition of said carbonate and/or bicarbonate reaction product. Where the downstream processing includes anaerobic treatment of the liquor, the pH of the liquor obtained following precipitation of the metals may be adjusted to 6.5 to 8, preferably about 7, by the addition of further mother liquor derived from the hydrogenation reaction.

Preferably the dissolution of the residue and subsequent precipitation of the metal catalyst components is effected by initially adding hydroxide to increase pH to a level such that the subsequent addition of the carbonate and/or bicarbonate is not accompanied by any substantial evolution of carbon dioxide. Suppression of carbon dioxide evolution is advantageous in order to avoid stripping volatiles such as acetic acid from the aqueous medium since the evolved gases/vapours would then need to be treated before disposal. Also, suppression of carbon dioxide evolution avoids operating and/or design problems, e.g. foaming and level control, in dealing with such evolution during the addition of the carbonate/bicarbonate ions.

Following precipitation and separation of the catalyst metal carbonates and/or bicarbonates, the liquor is conveniently subjected to anaerobic treatment or wet oxidation, optionally followed by aerobic treatment.

The invention will now be described by way of example only with reference to the accompanying drawings in which:
Figure 1 is a flow sheet illustrating the handling of mother liquor recycle in a process for the production of terephthalic acid; and
Figure 2 is a flow sheet illustrating a catalyst recovery system for use in conjunction with the process illustrated in Figure 1.

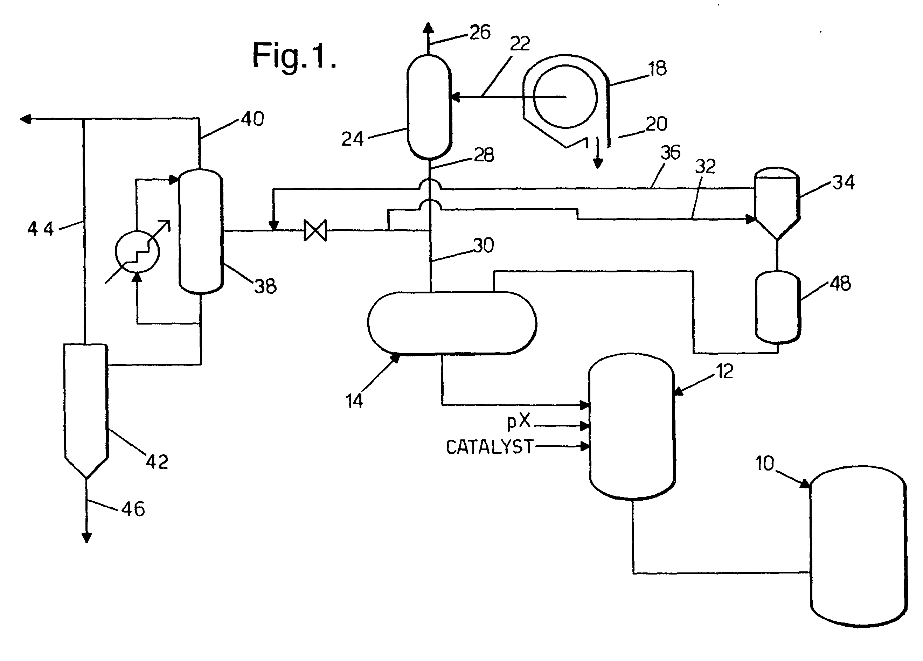

In the embodiment of Figure 1, terephthalic acid is produced in a reactor 10 by reacting p-xylene with oxygen (eg. air or oxygen enriched air) in acetic acid solvent containing some water and a dissolved catalyst system comprising heavy metals, usually cobalt and manganese, and bromine as a promoter. The p-xylene, acetic acid and catalyst may be supplied to the reactor via a feed mix drum 12 in which these components are mixed with recycled mother liquor from mother liquor drum 14. The oxygen/air is introduced separately into the reactor 10 via a feed line or lines (not shown). Further details of the reaction are given in our prior European Patent Applications Nos. 498591 and 502628. Typically the reaction is carried out at a temperature of 170-230°C and a pressure of several kg/cm² to 100 kg/cm², eg. 8-30 kg/cm².

The terephthalic acid is withdrawn from the reactor 10 in the form of a slurry of terephthalic acid crystals in mother liquor comprising acetic acid and some water. The slurry is then subjected to crystallisation in one or more crystallisation vessels (not shown) by reducing pressure and temperature so as to precipitate further terephthalic acid. Following the crystallisation process, the slurry is typically at a temperature of the order of 70 to 200°C. The slurry next undergoes an integrated solids-liquid separation process in which the crystals are separated from the mother liquor by filtration and are washed using water or acetic acid as the wash medium. The solids-liquid separation process is carried out in unit 18 under pressure using a filter medium across which a pressure differential is produced to effect displacement of the mother liquor and the wash liquor through the filter cake, comprising terephthalic acid crystals, which develops on the filter medium. The pressure differential may be produced by pressurising the upstream side of the filter medium with a gas or vapour or by hydraulically pressurising the slurry and the wash liqour. The integrated filtration and washing process may be carried out using for example a belt filter as disclosed in European Patent Application No. 502628 under conditions described therein or using a rotary suction filter or a pressure drum filter such as a BHS-Fest drum filter or a centrifuge. In the illustrated embodiment, the filtration and washing process is shown carried out in a rotary filter unit, if desired with countercurrent washing of the filter cake with water. The filter cake comprising terephthalic acid crystals is removed from the unit 18 via line discharge outlet 20 for further processing, e.g. preparation for use in polyester production without any further purification or purification to reduce the level of impurities therein followed by subsequent use in the production of polyesters, e.g. as disclosed in our prior International Patent Application No. WO93/24440.

The mother liquor filtrate derived from the solids-liquid separation unit 18 via line 22 largely consists of acetic acid (typically 85 - 95% by weight) and water (typically 5 - 15% by weight). The mother liquor also contains soluble organic by-products and intermediates produced in the reaction, reaction catalyst and residual terephthalic acid. Also with this type of filter, the wash liquor often mixes with the mother liquor stream. The recovered mother liquor is fed to a separator 24 in which the liquor is separated from the gas used to provide the pressure difference for the filtration and washing unit 18 (e.g. nitrogen). The gas is recovered via line 26 and the mother liquor via line 28. The mother liquor is split into two fractions, one of which is recycled via line 30 and mother liquor drum 14 back to the reactor and the second of which is purged from the process via line 32 in order to maintain the level of impurities in the system within acceptable limits. The mother liquor recycle fraction is typically in the range 0.7 to 0.99 (e.g. 0.7 to 0.95) and the purge fraction is correspondingly 0.3 to 0.01 (e.g. 0.3 to 0.05).

In contrast with conventional practice, before being subjected to concentration, the purge stream 32 is fed to a filter unit 34 which may comprise one or more candle-type filters within a pressure vessel. The candle-type filter unit 34 is designed so as to separate terephthalic acid fines. A particularly suitable filter unit for this duty is a Cricketfilter (Model 1200W-25/1500-100) which is a synthesis of conventional leaf filters and candle filters and is manufactured by Amafilter b.v., P O Box 396, 1800 AJ Alkmaar, Kwakelkade 28, 1823 CL. Alkmaar, Holland. Effective filtration was secured by using a PTFE filter cloth material in the Cricketfilter.

The filtered mother liquor purge is fed via line 36 to a stripper stillpot 38 in which a substantial part of the solvent (acetic acid) is boiled off and fed to an acetic acid recovery process (distillation column) via line 40. The residual liquor is fed to evaporator 42 for concentration. In evaporator 42, further acetic acid is driven off via line 44 for feed to acetic acid recovery in such a way as to leave the evaporator bottoms in a fluid state for supply of the resulting residue to a waste treatment system via line 46. In the process of the present invention, an increased amount of acetic acid can be recovered compared with the level of recovery achieved in the absence of filtering the mother liquor purge fraction. For instance, whereas 99.515% of the acetic acid was recovered in the absence of such filtration, in practice it has been found possible to increase this figure to 99.758% as a result of filtering the mother liquor purge fraction. As mentioned previously, the residue obtained from the evaporator may be disposed of or may be subjected to treatment by means of incineration or other treatment such as catalyst recovery or biological processing.

Periodically, the filter cake comprising terephthalic acid fines and some solid phase catalyst, is removed from unit 34 by backflushing with mother liquor, fresh acetic acid solvent or inert gas and is supplied to the mother liquor drum 14, or elsewhere, for recycle to the reactor 10. If desired, and as illustrated, the filter cake may first of all be reslurried in reslurry drum 48 using mother liquor or fresh acetic acid. During backflushing of the filter unit 34, the mother liquor supplied via line 32 may be diverted temporarily to the mother liquor drum 14 or bypass the filter and pass directly to the stripper stillpot 38.

A commercial plant producing terephthalic acid at the rate of 55 to 56 te/hr and operating with a mother liquor recycle of 85% was equipped with the Cricketfilter unit mentioned above and a comparison was made between plant operation with the Cricketfilter unit on-line and off-line. With the Cricketfilter unit off-line, the acetic acid loss via the mother liquor purge was 34 kg/hr and the rate of organic residue production was 5.04 kg of residue per tonne of terephthalic acid produced. With the Cricketfilter on-line, the acetic acid loss reduced to 23 kg/hr while the organic residue production rate fell to 4.13 kg of residue per tonne of terephthalic acid produced by the plant. Also, with the Cricketfilter on-line, the amount of terephthalic acid recovered from the mother liquor purge was about 70 kg/hr, which equates to a reduction in paraxylene feedstock to the process of about 0.13% - which represents significant annual cost savings.

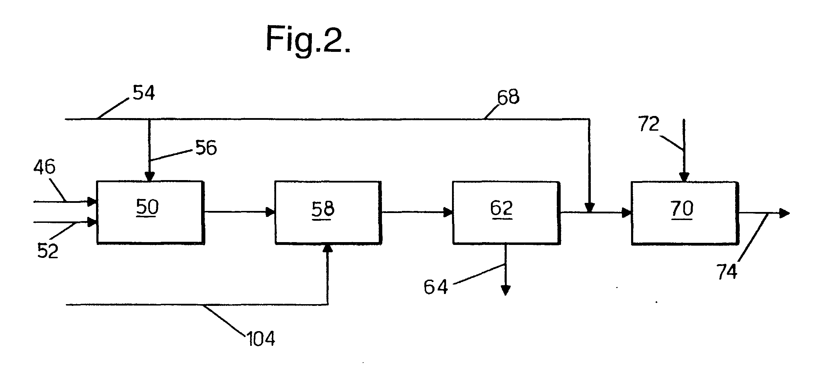

Referring to Figure 2, the residue obtained from the evaporator 42 is fed via line 46 to a stirred tank 50 together with a 5% caustic soda solution supplied via line 52 and an aqueous medium supplied via lines 54, 56. Although in Figure 2, the various components are shown as being fed separately to the tank 50, the residue may be slurried up in a portion of the aqueous medium in a slurry receiver upstream of the tank 50. At least a major part of the aqueous medium is advantageously constituted by mother liquor derived from plant for purifying crude terephthalic acid by hydrogenation of an aqueous solution of the crude acid in the presence of a noble metal catalyst such as platinum and/or palladium on an inert, e.g. carbon, support. Suitable plant for purifying crude terephthalic acid is described in EP-A-498591, EP-A-502628 and WO-A-93/24440, the entire disclosures of which are incorporated herein by this reference. As described in these prior patent publications, following hydrogenation the solution is passed through a crystallisation train resulting in a slurry of purified terephthalic acid crystals in aqueous mother liquor and the slurry is filtered and washed. The mother liquor filtrate (primary mother liquor) obtained may be used as the aqueous medium supplied to the tank 50. Alternatively, the primary mother liquor may be subjected to cooling or evaporation to precipitate further, but less pure, terephthalic acid crystals which, following separation from the secondary mother liquor, may be slurried in acetic acid for recycle to the oxidation reactor. The secondary mother liquor so obtained may then be used as the aqueous medium in the catalyst recovery system. If desired, the aqueous medium may comprise both primary and secondary mother liquor. The advantage of using the secondary mother liquor is that its organic content is reduced compared with the primary mother liquor. Typically the mother liquor supplied to the tank will comprise primarily water but will also contain small amounts of water, acetic acid, benzoic acid, paratoluic acid, terephthalic acid and manganese and cobalt acetates.

In the tank 50, at a temperature of about 60°C, 5% caustic soda is added to raises the pH to for example about 5.5 and the metals and organics are dissolved. The liquor obtained overflows into a precipitation tank 58 via a baffled outlet to prevent carryover of any solids still undergoing dissolution in tank 50. Sodium carbonate and/or bicarbonate obtained from a scrubber as described below is also supplied to the tank 58 via line 104. the rate of supply being such that the pH is raised to about 6.5 to about 9 leading to precipitation of the catalyst metals, primarily as carbonates and/or bicarbonates thereof. As mentioned previously, some precipitation of the metals as oxides (especially manganese oxides and/or hydroxides) may also occur especially if caustic soda is used in tank 50. The oxides are considered to be contaminants and consequently it may be preferred to substitute at least part of the caustic soda in tank 50 with sodium carbonate and/or bicarbonate derived from the same source as that supplied via line 104.

The contents of the precipitation tank 58 are passed to solids-liquid separator unit 62 which may, for instance, comprise a clarifier producing a solids-containing underflow and a liquor overflow. The underflow is pumped to a sludge buffer tank (not shown) and subsequently passed to a filter press to produce a relatively dry cake containing the catalyst metal carbonates and/or bicarbonates. The catalyst metals recovered in this way may be recycled via line 64 to the oxidation reactor 10 as their carbonates and/or bicarbonates or, alternatively, before recycle they may be converted to for example acetates by reaction with acetic acid. The unit 62 may alternatively comprise a centrifuge.

The overflow 66 from the clarifier is mixed with additional mother liquor supplied via lines 54, 68 and passes to a final neutralisation tank 70 to which caustic soda is added via line 72 in order to adjust the pH of the liquor prior to feed to downstream processing plant via line 74. The mother liquor supplied via line 54 typically corresponds to the amount which is to be purged from the purification plant to maintain the levels of impurities within acceptable limits, especially when the mother liquor is to be recycled in the manner disclosed in EP-A-498591, EP-A-502628 and WO-A-93/24440. The purged mother liquor requires treatment before disposal because of its COD and such treatment will usually entail adjustment of its pH.

It will be seen that the process described with reference to Figure 2 allows the purge to be employed as a vehicle for recovery of catalyst metals despite the organic content of the mother liquor purge. Rather than pass the entire amount of mother liquor purge to the residue dissolution tank 50, it is preferably divided into two fractions, as indicated by lines 56 and 58, so that the equipment size and cost in these stages can be reduced. Another factor governing the amount of mother liquor employed in the dissolution stage(tank 50) is the evolution of CO₂ that occurs in the course of increasing pH in the dissolution stage. If the sodium (or other alkali metal) carbonate and/or bicarbonate is added at low pH levels, for a given amount of the liquor present, the amount of CO₂ that can remain in solution (and hence be available as carbonate ions at the precipitation stage) is reduced compared with addition at higher pH levels.

Consequently to avoid loss of CO₂ from solution on introduction of the sodium carbonate and/or bicarbonate, it is desirable to secure conditions which suppress CO₂ evolution from the solution.This can be achieved by controlling pH (e.g. a pH of about 5.5 is suitable) and/or the level of dilution during the dissolution process. Whilst equipment size and cost is a factor which implies minimising the amount of mother liquor used in the dissolution stage, it will be generally desirable to employ sufficient mother liquor consistent with suppressing CO₂ evolution.

The neutralisation carried out in tank 70 will usually involve adjustment of pH within the range 6.5 to 8, preferably 7, for compatability with the downstream processing of the liquor. Such downstream processing may take various forms such as anaerobic treatment (e.g. using the UASB process - upflow anaerobic sludge blanket) followed by aerobic treatment (e.g. activated sludge treatment), or wet oxidation using for example the known ZIMPRO or LOPROX processes.

The carbonate used in the treatment of the residue may be derived from a scrubber for scrubbing effluent gas from plant for the production of terephthalic acid after treatment of the effluent gas by high temperature combustion, preferably in the presence of a catalyst and under elevated pressure, to convert methyl bromide in the effluent to bromine and/or hydrogen bromide. A process for the treatment of the effluent gas, including scrubbing thereof, is described in our published International Patent Application No. WO 96/39595, the entire disclosure of which is incorporated herein by this reference. The scrubbing liquid is preferably caustic soda, which is converted to sodium carbonate and bicarbonate in the scrubbing vessel as a result of absorption into the hydroxide of carbon dioxide contained in the effluent gas. The sodium (bi)carbonate resulting from the scrubbing process is then used in the recovery of catalyst metals as described above thereby making efficient use of the scrubbing liquor.

## Claims

1. A process for the production of an aromatic carboxylic acid comprising oxidising a precursor of the aromatic carboxylic acid in an aqueous liquid phase medium comprising acetic acid and in the presence of a heavy metal catalyst system, withdrawing from the reaction mixture a slurry of the aromatic carboxylic acid in mother liquor comprising mainly the acetic acid, subjecting the slurry to a solids-liquid separation to recover crystals of aromatic carboxylic acid, recycling a first fraction of the resulting mother liquor to the oxidation reaction, concentrating a second fraction of the separated mother liquor to remove acetic acid, and disposing of or processing the concentrated residue, characterised in that the second fraction of mother liquor is subjected to solids-liquid separation prior to concentration thereof.

2. A process as claimed in Claim 1 in which the solids-liquid separation is effective to recover from 50% up to 80% by weight of the solids present in said second fraction.

3. A process as claimed in Claim 1 or 2 in which at least part of the aromatic carboxylic acid recovered from the second fraction is recycled to the oxidation reaction.

4. A process as claimed in Claim 3 in which the aromatic carboxylic acid is recycled by reslurrying it with solvent and feeding the slurry to the reactor.

5. A process as claimed in Claim 4 in which the solvent used to reslurry said aromatic carboxylic acid comprises mother liquor derived from the oxidation reaction.

6. A process as claimed in Claim 4 in which the solvent used to reslurry said aromatic carboxylic acid comprises solvent recovered during concentration of the second fraction.

7. A process as claimed in any one of Claims 1 to 6 in which at least part of the aromatic carboxylic acid recovered from said second fraction is combined with aromatic carboxylic acid recovered from the slurry obtained following the oxidation reaction and primary solids-liquid separation.

8. A process as claimed in any one of Claims 1 to 7 in which at least part of the aromatic carboxylic acid recovered from said second fraction is introduced into a crystallisation process following the oxidation reaction and preceding the primary solids-liquid separation.

9. A process as claimed in any one of Claims 1 to 8 in which the recovered aromatic carboxylic acid is recycled to the oxidation reaction together with aromatic carboxylic acid recovered from elsewhere in the process.

10. A process as claimed in any one of Claims 1 to 9 in which solvent recovered from concentration of the mother liquor second fraction is recycled to the oxidation reaction.

11. A process as claimed in any one of the preceding claims in which the solids-liquid separation of said mother liquor second fraction is effected by means of filtration.

12. A process as claimed in Claim 11 in which the filter is one in which build up of a filter cake on a filter medium occurs in such a way that the filter cake is itself instrumental in filtering the solids-containing liquid.

13. A process as claimed in Claim 12 in which the filtration is effected by means of one or more candle filters.

14. A process as claimed in any one of Claims 1 to 13 in which the concentrated residue including a substantial part of its organic content is dissolved in an aqueous medium and the metal catalyst components are precipitated from the solution.

15. A process as claimed in Claim 14 in which the metal catalyst components are precipitated from said solution by the inclusion of metal salt-forming anions in the solution.

16. A process as claimed in Claim 14 or 15 in which the aromatic carboxylic acid is one having a solubility in water less than 1% by weight at 25°C.

17. A process as claimed in any one of Claims 14 to 17 in which substantially the whole of the residue is dissolved in said aqueous medium.

18. A process as claimed in any one of Claims 14 to 17 in which the catalyst metals are precipitated from the aqueous medium by the inclusion of carbonate and/or bicarbonate ions to the medium.

19. A process as claimed in Claim 18 in which the catalyst metals are precipitated by the inclusion in the aqueous medium of the carbonate and/or bicarbonate reaction product obtained following contacting a metal or ammonium hydroxide with a carbon dioxide-containing offgas derived from the oxidation reaction in which said aromatic carboxylic acid is produced.

20. A process as claimed in any one of Claims 14 to 19 in which the aqueous medium comprises, as at least a major component thereof, an organic material-containing mother liquor derived from the hydrogenation of an aqueous solution of the aromatic carboxylic acid.

21. A process as claimed in any one of Claims 14 to 20 in which solubilisation of substantially all of the residue in the aqueous medium is effected by inclusion of an alkaline agent added to the aqueous medium prior to and/or in the course of combining the residue with the aqueous medium.

22. A process as claimed in any one of Claims 18 or 19 in which the dissolution of the residue and subsequent precipitation of the metal catalyst components is effected by initially adding hydroxide to increase pH to a level such that the subsequent addition of the carbonate and/or bicarbonate is not accompanied by any substantial evolution of carbon dioxide.

23. A process as claimed in any one of Claims 1 to 22 in which the aromatic carboxylic acid comprises terephthalic acid.

## Patentansprüche

1. Verfahren für die Herstellung einer aromatischen Carbonsäure, umfassend Oxidieren einer Vorstufe der aromatischen Carbonsäure in dem Medium einer wäßrigen flüssigen, Essigsäure umfassenden Phase und in Anwesenheit eines Schwermetallkatalysatorsystems, Abziehen einer Aufschlämmung der aromatischen Carbonsäure in hauptsächlich die Essigsäure umfassender Mutterlösung aus dem Reaktionsgemisch, Unterwerfen der Aufschlämmung einer Trennung von Feststoffen und Flüssigkeit, um die Kristalle der aromatischen Carbonsäure zu gewinnen, Rückführen einer ersten Fraktion der entstehenden Mutterlösung in die Oxidationsreaktion, Einengen einer zweiten Fraktion der abgetrennten Mutterlösung, um Essigsäure zu entfernen, und Verwerfen oder Verarbeiten des eingeengten Rückstandes, dadurch gekennzeichnet, daß die zweite Fraktion der Mutterlösung vor ihrer Einengung der Trennung von Feststoffen und Flüssigkeit unterworfen wird.

2. Verfahren nach Anspruch 1, bei dem die Trennung von Feststoffen und Flüssigkeit wirksam ist, um 50 Gew.-% bis zu 80 Gew.-% der Feststoffe, die in der zweiten Fraktion vorhanden sind, zu gewinnen.

3. Verfahren nach Anspruch 1 oder 2, bei dem zumindest ein Teil der aromatischen Carbonsäure, die aus der zweiten Fraktion gewonnen wird, in die Oxidationsreaktion zurückgeführt wird.

4. Verfahren nach Anspruch 3, bei dem die aromatische Carbonsäure zurückgeführt wird, indem sie mit Lösungsmittel wiederaufgeschlämmt wird und die Aufschlämmung in den Reaktor geführt wird.

5. Verfahren nach Anspruch 4, bei dem das Lösungsmittel, das verwendet wird, um die aromatische Carbonsäure wiederaufzuschlämmen, Mutterlösung umfaßt, die aus der Oxidationsreaktion erhalten wird.

6. Verfahren nach Anspruch 4, bei dem das Lösungsmittel, das verwendet wird, um die aromatische Carbonsäure wiederaufzuschlämmen, Lösungsmittel umfaßt, das während der Einengung der zweiten Fraktion gewonnen wird.

7. Verfahren nach einem der Ansprüche 1 bis 6, bei dem zumindest ein Teil der aromatischen Carbonsäure, die aus der zweiten Fraktion gewonnen wird, mit der aromatischen Carbonsäure vereinigt wird, die aus der im Anschluß an die Oxidationsreaktion und die primäre Trennung von Feststoffen und Flüssigkeit erhaltenen Aufschlämmung gewonnen wird.

8. Verfahren nach einem der Ansprüche 1 bis 7, bei dem zumindest ein Teil der aromatischen Carbonsäure, die aus der zweiten Fraktion gewonnen wird, im Anschluß an die Oxidationsreaktion und vor der primären Trennung von Feststoffen und Flüssigkeit in ein Kristallisationsverfahren eingeführt wird.

9. Verfahren nach einem der Ansprüche 1 bis 8, bei dem die gewonnene aromatische Carbonsäure zusammen mit der aromatischen Carbonsäure, die von anderswo in dem Verfahren gewonnen wird, in die Oxidationsreaktion zurückgeführt wird.

10. Verfahren nach einem der Ansprüche 1 bis 9, bei dem das Lösungsmittel, das aus der Einengung der zweiten Fraktion der Mutterlösung gewonnen wird, in die Oxidationsreaktion zurückgeführt wird.

11. Verfahren nach einem der vorhergehenden Ansprüche, bei dem die Trennung von Feststoffen und Flüssigkeit der zweiten Fraktion der Mutterlösung mittels Filtration ausgeführt wird.

12. Verfahren nach Anspruch 11, bei dem das Filter eines ist, bei dem der Aufbau eines Filterkuchens auf einem Filtermedium in einer derartigen Weise vor sich geht, daß der Filterkuchen selbst zum Filtrieren der Feststoffe enthaltenden Flüssigkeit beiträgt.

13. Verfahren nach Anspruch 12, bei dem die Filtration mittels eines oder mehrerer Kerzenfilter ausgeführt wird.

14. Verfahren nach einem der Ansprüche 1 bis 13, bei dem der eingeengte Rückstand einschließlich eines wesentlichen Teils seines organischen Gehalts in einem wäßrigen Medium gelöst wird und die Metallkatalysatorkomponenten aus der Lösung ausgefällt werden.

15. Verfahren nach Anspruch 14, bei dem die Metallkatalysatorkomponenten durch den Einschluß von Metallsalz erzeugenden Anionen in die Lösung aus der Lösung ausgefällt werden.

16. Verfahren nach Anspruch 14 oder 15, bei dem die aromatische Carbonsäure eine ist, die eine Löslichkeit in Wasser von weniger als 1 Gew.-% bei 25°C aufweist.

17. Verfahren nach einem der Ansprüche 14 bis 17, bei dem im wesentlichen der gesamte Rückstand in dem wäßrigen Medium gelöst wird.

18. Verfahren nach einem der Ansprüche 14 bis 17, bei dem die Katalysatormetalle durch den Einschluß von Carbonat- und/oder Bicarbonationen in das Medium aus dem wäßrigen Medium ausgefällt werden.

19. Verfahren nach Anspruch 18, bei dem die Katalysatormetalle durch den Einschluß des Carbonat- und/oder Bicarbonatreaktionsprodukts, das im Anschluß an das Inkontaktbringen eines Metall- oder Ammoniumhydroxids mit einem Kohlendioxid enthaltenden Abgas, das aus der Oxidationsreaktion erhalten wird, bei der die aromatische Carbonsäure hergestellt wird, in das wäßrige Medium ausgefällt werden.

20. Verfahren nach einem der Ansprüche 14 bis 19, bei dem das wäßrige Medium als zumindest eine Hauptkomponente davon eine organisches Material enthaltende Mutterlösung, erhalten aus der Hydrierung einer wäßrigen Lösung der aromatischen Carbonsäure, umfaßt.

21. Verfahren nach einem der Ansprüche 14 bis 20, bei dem die Solubilisierung im wesentlichen des gesamten Rückstandes in dem wäßrigen Medium durch Einschluß eines alkalischen Mittels, hinzugesetzt zu dem wäßrigem Medium vor und/oder im Verlauf des Vereinigen des Rückstands mit dem wäßrigen Medium, ausgeführt wird.

22. Verfahren nach einem der Ansprüche 18 oder 19, bei dem die Auflösung des Rückstandes und nachfolgende Ausfällung der Metallkatalysatorkomponenten durch anfängliches Hinzusetzen von Hydroxid ausgeführt wird, um den pH auf ein derartiges Niveau zu vergrößern, daß die nachfolgende Zugabe des Carbonats und/oder Bicarbonats nicht von einer wesentlichen Entwicklung von Kohlendioxid begleitet wird.

23. Verfahren nach einem der Ansprüche 1 bis 22, bei dem die aromatische Carbonsäure Terephthalsäure umfaßt.

## Revendications

1. Procédé pour la production d'un acide carboxylique aromatique, comprenant l'oxydation d'un précurseur de l'acide carboxylique aromatique dans un milieu en phase liquide aqueuse comprenant de l'acide acétique et en présence d'un système catalytique à métal lourd, le soutirage, à partir du mélange réactionnel, d'une suspension épaisse de l'acide carboxylique aromatique dans une liqueur mère comprenant principalement l'acide acétique, la soumission de la suspension épaisse à une séparation solides-liquide pour recueillir des cristaux de l'acide carboxylique aromatique, le recyclage d'une première fraction de la liqueur mère résultante à la réaction d'oxydation, la concentration d'une seconde fraction de la liqueur mère séparée pour éliminer l'acide acétique, et l'élimination ou le traitement du résidu concentré, caractérisé en ce que la seconde fraction de liqueur mère est soumise à une séparation solides-liquide avant la concentration de celle-ci.

2. Procédé selon la revendication 1, dans lequel la séparation solides-liquide permet de recueillir de 50% jusqu'à 80% en poids des solides présents dans ladite seconde fraction.

3. Procédé selon la revendication 1 ou 2, dans lequel au moins une partie de l'acide carboxylique aromatique recueilli à partir de la seconde fraction est recyclé à la réaction d'oxydation.

4. Procédé selon la revendication 3, dans lequel l'acide carboxylique aromatique est recyclé en le remettant en suspension épaisse avec un solvant et en introduisant la suspension épaisse dans le réacteur.

5. Procédé selon la revendication 4, dans lequel le solvant utilisé pour remettre ledit acide carboxylique aromatique en suspension épaisse comprend une liqueur mère provenant de la réaction d'oxydation.

6. Procédé selon la revendication 4, dans lequel le solvant utilisé pour remettre ledit acide carboxylique aromatique en suspension épaisse comprend un solvant récupéré pendant la concentration de la seconde fraction.

7. Procédé selon l'une quelconque des revendications 1 à 6, dans lequel au moins une partie de l'acide carboxylique aromatique récupéré de ladite seconde fraction est combiné avec l'acide carboxylique aromatique récupéré de la suspension épaisse obtenue après la réaction d'oxydation et la séparation solides-liquide primaire.

8. Procédé selon l'une quelconque des revendications 1 à 7, dans lequel au moins une partie de l'acide carboxylique aromatique récupéré de ladite seconde fraction est introduit dans un traitement de cristallisation après la réaction d'oxydation et avant la séparation solides-liquide primaire.

9. Procédé selon l'une quelconque des revendications 1 à 8, dans lequel l'acide carboxylique aromatique récupéré est recyclé à la réaction d'oxydation avec l'acide carboxylique aromatique récupéré d'autre part dans le procédé.

10. Procédé selon l'une quelconque des revendications 1 à 9, dans lequel le solvant récupéré par concentration de la seconde fraction de liqueur mère est recyclé à la réaction d'oxydation.

11. Procédé selon l'une quelconque des revendications précédentes, dans lequel la séparation solides-liquide de ladite seconde fraction de liqueur mère est effectuée au moyen d'une filtration.

12. Procédé selon la revendication 11, dans lequel le filtre est un filtre dans lequel une accumulation d'un gâteau de filtre sur un milieu filtrant se produit de sorte que le gâteau de filtre contribue lui-même à la filtration du liquide contenant des solides.

13. Procédé selon la revendication 12, dans lequel la filtration est effectuée au moyen d'un ou plusieurs filtres à bougie.

14. Procédé selon l'une quelconque des revendications 1 à 13, dans lequel le résidu concentré incluant une partie substantielle de sa teneur en substances organiques est dissous dans un milieu aqueux et les constituants catalytiques métalliques sont précipités de la solution.

15. Procédé selon la revendication 14, dans lequel les constituants catalytiques métalliques précipitent de ladite solution par l'inclusion d'anions formant un sel métallique dans la solution.

16. Procédé selon la revendication 14 ou 15, dans lequel l'acide carboxylique aromatique est un acide ayant une solubilité dans l'eau inférieure à 1 % en poids à 25 °C.

17. Procédé selon l'une quelconque des revendications 14 à 17, dans lequel substantiellement la totalité du résidu se dissout dans ledit milieu aqueux.

18. Procédé selon l'une quelconque des revendications 14 à 17, dans lequel les métaux catalytiques précipitent du milieu aqueux par l'inclusion d'ions carbonates et/ou bicarbonates au milieu.

19. Procédé selon la revendication 18, dans lequel les métaux catalytiques précipitent par l'inclusion, dans le milieu aqueux, du produit de réaction carbonate et/ou bicarbonate obtenu après avoir mis en contact un hydroxyde de métal ou d'ammonium avec un effluent gazeux contenant du dioxyde de carbone provenant de la réaction d'oxydation dans laquelle ledit acide carboxylique aromatique est produit.

20. Procédé selon l'une quelconque des revendications 14 à 19, dans lequel le milieu aqueux comprend, en tant qu'au moins un constituant majeur de celui-ci, une liqueur mère contenant une matière organique, provenant de l'hydrogénation d'une solution aqueuse de l'acide carboxylique aromatique.

21. Procédé selon l'une quelconque des revendications 14 à 20, dans lequel la solubilisation de substantiellement la totalité du résidu dans le milieu aqueux est effectuée par l'inclusion d'un agent alcalin ajouté au milieu aqueux avant et/ou au cours de la combinaison du résidu avec le milieu aqueux.

22. Procédé selon l'une quelconque des revendications 18 ou 19, dans lequel la dissolution du résidu et la précipitation subséquente des constituants catalytiques métalliques sont effectuées en ajoutant initialement un hydroxyde pour augmenter le pH à un niveau tel que l'addition subséquente du carbonate et/ou bicarbonate ne s'accompagne d'aucun dégagement substantiel de dioxyde de carbone.

23. Procédé selon l'une quelconque des revendications 1 à 22, dans lequel l'acide carboxylique aromatique comprend de l'acide téréphtalique.
